# EUROPEAN PATENT SPECIFICATION

(11) **EP 2 675 405 B1**
(45) Date of publication and mention of the grant of the patent: **10.02.2016**
(21) Application number: 12705279.3
(22) Date of filing: 16.02.2012
(51) Int. Cl.: A61F 5/00

(54) **HYDRAULIC GASTRIC BAND WITH REVERSIBLE SELF-OPENING MECHANISM**
HYDRAULISCHES MAGENBAND MIT UMKEHRBAREM SELBSTÖFFNENDEM MECHANISMUS
ANNEAU GASTRIQUE HYDRAULIQUE À MÉCANISME D'AUTO-OUVERTURE RÉVERSIBLE

(30) Priority: 17.02.2011 US 201113029409
(43) Date of publication of application: 25.12.2013
(73) Proprietor: APOLLO ENDOSURGERY, INC., Austin, TX 78746 (US)
(72) Inventor: FRIDEZ, Pierre, CH-1055 Froideville (CH); BERTOLOTE, Tiago, CH-1202 Geneva (CH); RAEMY, Xavier, CH-1092 Belmont-sur-Lausanne (CH); KAEGI, Fabian, CH-1012 Lausanne (CH); RION, Julien, CH-1110 Morges (CH)
(74) Representative: Hoffmann Eitle
(86) International application number: PCT/EP2012/052728
(87) International publication number: WO 2012/110623

(56) References cited:
- EP-A1- 2 191 796
- US-A1- 2009 054 914
- US-A1- 2010 185 049

## Description

### FIELD

The present invention generally relates to medical systems and appartus and uses thereof for treating obesity and/or obesity-related diseases, and more specifically, relates to gastric banding systems that self-adjust when an obstruction is present in a gastric lumen of a stomach of a patient.

### BACKGROUND

Adjustable gastric banding apparatus have provided an effective and substantially less invasive alternative to gastric bypass surgery and other conventional surgical weight loss procedures. Despite the positive outcomes of invasive weight loss procedures, such as gastric bypass surgery, it has been recognized that sustained weight loss can be achieved through a laparoscopically-placed gastric band, for example, the LAP-BAND^{®} (Allergan, Inc., Irvine, CA) gastric band or the LAP-BAND AP^{®} (Allergan, Inc., Irvine, CA) gastric band. Generally, gastric bands are placed about the cardia, or upper portion, of a patient's stomach forming a stoma that restricts the food's passage into a lower portion of the stomach. When the stoma is of an appropriate size that is restricted by a gastric band, the food held in the upper portion of the stomach may provide a feeling of satiety or fullness that discourages overeating.

Unlike gastric bypass procedures, gastric band apparatuses are reversible and require no permanent modification to the gastrointestinal tract. An example of a gastric banding system is disclosed in Roslin, et al., U.S. Patent Pub. No. 2006/0235448.

Over time, a stoma created by a gastric band may need adjustment in order to maintain an appropriate size, which is neither too restrictive nor too passive. Accordingly, prior art gastric band systems provide a subcutaneous fluid access port connected to an expandable or inflatable portion of the gastric band. By adding fluid to or removing fluid from the inflatable portion by means of a hypodermic needle inserted into the access port, the effective size of the gastric band can be adjusted to provide a tighter or looser constriction.

Birk, et al., U.S. Patent Pub. No. US 2010/0305397 discloses a hydraulic mechanical gastric band that includes an external control unit capable of communicating with a sensor to regulate the constriction of the band about the organ or the duct. The sensor sends data to the external control unit to control operations of the gastric band based on the data from the sensor. However, this application includes an external control unit to control operations of the gastric band.

Sometimes, adjustment of a gastric band may be desirable in between adjustments made by a physician. For example, during normal operation of the gastric band, the gastric band applies pressure to an outer surface of the upper stomach. But in some instances, the patient may swallow a bolus, or attempt to pass an obstruction (e.g., a large piece of food), that is too large to pass through the constriction produced by the gastric band. The result can be a painful experience which, if it persists, may require medical intervention to release the blockage.

Some attempts have been made to account for the possibility of a blockage. For example, Coe, et al., U.S. Patent Pub. No. 2009/0216255 discloses a flow control device that moves fluid between a hydraulic restriction system and a fluid source. The additional flow control device controls a rate of fluid flow between the restriction device and the fluid source. In addition, Coe, et al., European Patent Application No. 2 074 970 A1 discloses a separate restriction device and a pressure adjustment device. The pressure adjustment device regulates a constant force applied by the restriction device using, for example, a bellows and a spring.

Further, Lechner, U.S. Patent Pub. No. 2009/0054914 discloses a controllable stomach band that has a chamber for controlling restriction of the stomach band. The chamber is coupled to a separate pressure chamber that receives fluid leaving the chamber in the stomach band. The pressure chamber is separated from the esophageal-gastric junction of the patient's stomach. The pre-amble of claim 1 is based on this document.

Further, Steffen, U.S. Patent Pub. No. 2009/0062826 discloses an adjustable gastric band with a "conveyance device" that is powered by a "power storage device." The power storage device operates the conveyance device to move fluid between expandable chambers to adjust the gastric band.

Some attempts have been made to account for the possibility of blockage (e.g., by a bolus of food). For example, Snow, et al., U.S. Application Serial No. 12/770,617 discloses a self-adjusting gastric band that temporarily and automatically opens up to allow a bolus through. However, this application does not utilize complicated fluid control mechanisms, flow rate limiting devices, and/or valves to regulate the transfer of fluid within the self-adjusting gastric band.

### SUMMARY

Accordingly, it is desirable to develop a gastric banding system that is capable of providing needed pressure to the stomach, yet is also capable of adapting and opening up to allow an obstruction to pass through a portion of the stomach being constricted. A reversible self-opening mechanism, or adjustment system, may be utilized to allow an obstruction to pass through a portion of a gastric lumen constricted by a gastric band. The adjustment system may allow the gastric band to open quickly in response to the obstruction passing through the gastric lumen, yet may also allow the gastric band to slowly return to the size the gastric band had before the obstruction was present.

In one embodiment, the present invention comprises a system including a gastric band having an inflatable member configured to contain fluid and apply constriction to a portion of a gastric lumen of the stomach, the inflatable member being moveable from a constricted state to a passage state for allowing an obstruction to pass through a portion of the gastric lumen, the passage state being when less fluid is contained in the inflatable member than in the constricted state. A valve is configured to move from a closed position to an open position when a pressure of the fluid from the inflatable member increases over a threshold in response to the obstruction passing through the gastric lumen, and to allow the fluid from the inflatable member to pass through the valve at a first flow rate. A reservoir is configured to receive the fluid passed through the valve, allowing the inflatable member to move from the constricted state to the passage state, and allowing the obstruction to pass through the portion of the gastric lumen. A flow restriction device is configured to allow the fluid received by the reservoir to pass from the reservoir to the inflatable member at a second flow rate that is less than the first flow rate, allowing the inflatable member to return to the constricted state.

In one embodiment, the valve and the flow restriction device are contained within an access port housing. A reservoir is coupled to the access port housing. The reservoir is configured to receive fluid from the gastric band automatically when an obstruction passes through the gastric lumen. The obstruction creates a force that is large enough to open the valve, which causes fluid to pass from the gastric band to the reservoir at a faster flow rate. Once the obstruction has passed through the constricted portion of the gastric lumen, the pressure of the reservoir is greater than that of the gastric band, and fluid then flows back to the gastric band through the flow restriction device, at a slower flow rate. The flow restriction device allows the gastric band to slowly return to an equilibrium size, or the size the gastric band had before the obstruction was present. The slow return of the gastric band to its equilibrium size prevents wear on the gastric band, and prevents damage to local tissues. The threshold opening pressure of the valve allows the gastric band to substantially maintain its size up to the threshold pressure, enhancing the therapeutic success of the gastric band.

In one embodiment, the present invention comprises a system including a gastric band having an inflatable member configured to contain fluid and apply constriction to a portion of a gastric lumen of the stomach, the inflatable member being moveable from a constricted state to a passage state for allowing an obstruction to pass through a portion of the gastric lumen, the passage state being when less fluid is contained in the inflatable member than in the constricted state. A valve is configured to move from a closed position to an open position when a pressure of the fluid in the inflatable member increases over a threshold in response to the obstruction passing through the gastric lumen, the valve in the open position allowing fluid to pass through the valve at a first flow rate. A reservoir is configured to receive fluid when the valve is in the open position, causing the inflatable member to move from the constricted state to the passage state, and allowing the obstruction to pass through the portion of the gastric lumen. A flow restriction device is configured to pass fluid through the flow restriction device at a second flow rate that is less than the first flow rate, allowing the inflatable member to return to the constricted state from the passage state.

In one embodiment, a shunt valve is incorporated into the gastric banding system. The shunt valve allows a physician to vary an amount of fluid in the gastric banding system without having to pass fluid through the flow restriction device.

In one embodiment, an asymmetric flow regulator, including a valve that is configured to open to allow fluid from the gastric band to pass through the valve, when a pressure of the fluid exceeds a threshold, and a flow restriction device, is utilized. The asymmetric flow regulator is positioned in series with a reservoir and an access port.

In one embodiment, an asymmetric flow regulator, including a valve that is configured to open to allow fluid from the gastric band to pass through the valve, when a pressure of the fluid exceeds a threshold, and a flow restriction device, is utilized. The asymmetric flow regulator is positioned in parallel with a reservoir and an access port.

### BRIEF DESCRIPTION OF THE DRAWINGS

FIG. 1 illustrates a perspective view of a gastric banding system according to an embodiment of the present invention.
FIG. 2 illustrates a perspective view of a gastric band according to an embodiment of the present invention.
FIG. 3 illustrates a perspective view of a gastric band according to an embodiment of the present invention.
FIG. 4 illustrates a perspective view of a gastric banding system according to an embodiment of the present invention.
FIG. 5A illustrates a schematic view of a gastric banding system according to an embodiment of the present invention.
FIG. 5B illustrates a schematic view of the gastric banding system in the state shown in FIG. 5A according to an embodiment of the present invention.
FIG. 6A illustrates a schematic view of a gastric banding system according to an embodiment of the present invention.
FIG. 6B illustrates a schematic view of the gastric banding system in the state shown in FIG. 6A according to an embodiment of the present invention.
FIG. 7A illustrates a schematic view of a gastric banding system according to an embodiment of the present invention.
FIG. 7B illustrates a schematic view of the gastric banding system in the state shown in FIG. 7A according to an embodiment of the present invention.
FIG. 8A illustrates a schematic view of a gastric banding system according to an embodiment of the present invention.
FIG. 8B illustrates a schematic view of the gastric banding system in the state shown in FIG. 8A according to an embodiment of the present invention.
FIG. 9 illustrates a side cross-section view of an access port according to an embodiment of the present invention.
FIG. 10 illustrates a side cross-section view of an access port according to an embodiment of the present invention.
FIG. 11 illustrates a perspective view of a gastric banding system according to an embodiment of the present invention.
FIG. 12 illustrates a schematic view of the gastric banding system shown in FIG. 11 according to an embodiment of the present invention.
FIG. 13 illustrates a perspective view of a gastric banding system according to an embodiment of the present invention.
FIG. 14 illustrates a schematic view of the gastric banding system shown in FIG. 13 according to an embodiment of the present invention.

### DETAILED DESCRIPTION

The present invention generally provides for gastric banding systems, for example, systems for the treatment of obesity and obesity related conditions, as well as systems that allow adjustment of gastric bands in response to an obstruction passing through a gastric lumen.

The present invention provides for an adjustment system that automatically increases the size of a constricted portion of a gastric lumen in response to an obstruction passing through the gastric lumen. The size of the constricted portion of the gastric lumen increases rapidly when the obstruction approaches a gastric band that constricts the lumen. The adjustment system is also configured to slowly return the constricted gastric lumen to an equilibrium size the lumen had, prior to the obstruction passing through the lumen.

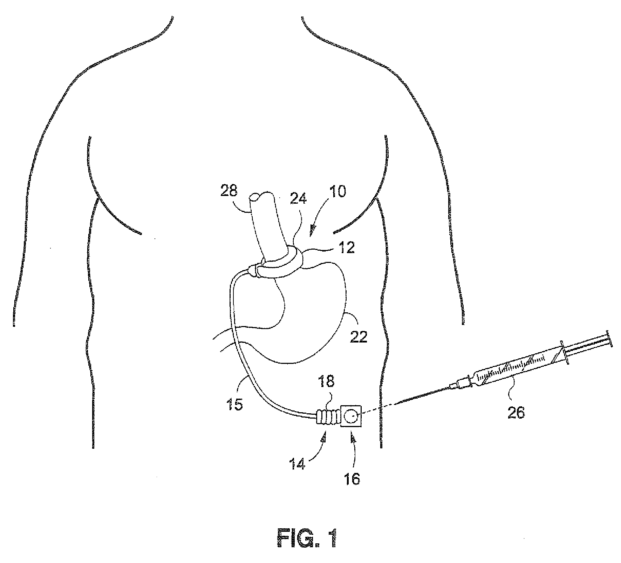

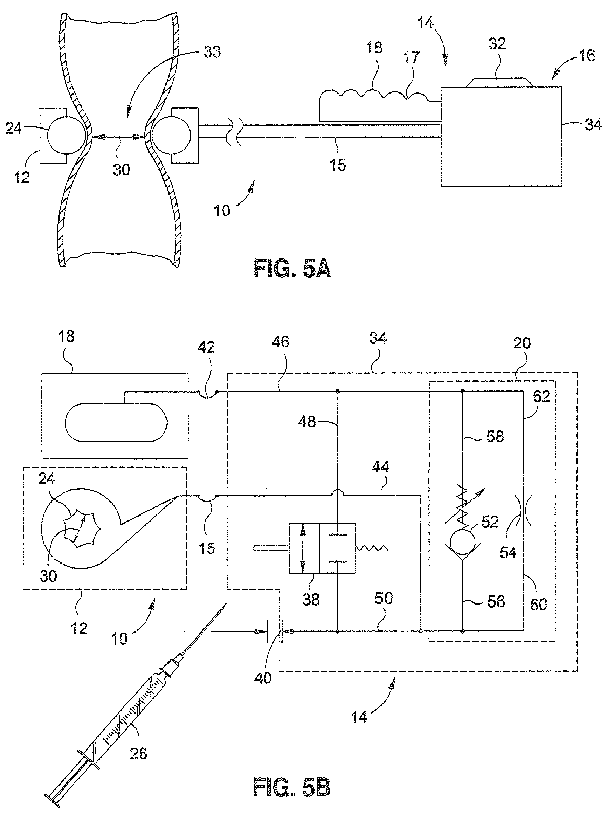

FIG. 1 illustrates an embodiment of the present invention including a gastric banding system **10** comprising a gastric band **12**, an adjustment system **14**, and a tube **15** connecting the gastric band **12** to the adjustment system **14**. In the embodiment shown in FIG. 1, the adjustment system **14** includes an access port **16**, a reservoir **18**, and an asymmetric flow regulator **20** (visible in FIG. 5B) contained within the access port **16**.

The gastric band **12** comprises a strap-like member capable of encircling a portion of a patient's stomach **22** to form a stoma. The gastric band **12** is preferably a hydraulic gastric band, having an inflatable member **24** that is filled with a fluid, such as saline. The inflatable member **24** serves as a cuff or a ring around a portion of the patient's stomach, which constricts the stomach, to a degree, based on the amount of fluid in the inflatable member **24**.

The access port **16** comprises an implantable device that is used by a physician to inflate the inflatable member **24** of the gastric band **12**. The access port **16** is configured to be fixed subcutaneously within the patient's body. The access port **16** is preferably fixed to the patient's muscle wall. The access port **16** is fixed to the muscle wall through sutures, anchors, tacks, or the like. The access port **16** is capable of receiving a syringe **26** that is inserted by a physician to transfer fluid to and from the inflatable member **24** of the gastric band **12.** The fluid passes between the access port **16** and the inflatable member **24** through the tube **15.**

The gastric band **12** is inserted into the patient's body laparoscopically. During laparoscopic insertion of the gastric band **12**, the gastric band **12** is wrapped around the portion of the patient's body to be constricted, and is then secured in position. The inflatable member **24** contacts the portion of the patient's stomach to be constricted. The gastric band **12** preferably encircles the cardia, or esophageal junction, of the patient's stomach. After the gastric band **12** has been fixed around a portion of the patient's stomach, the access port **16** is fixed to the patient's muscle wall. The tube **15** is then connected from the inflatable member **24** of the gastric band **12** to the access port **16**. A physician will then inject the access port **16** with an amount of fluid, as desired, to inflate the inflatable member **24** to an appropriate degree or size, depending on the physical characteristics of the patient, and the desired treatment plan for the patient. For example, if the patient is severely obese, then a greater degree of restriction and a greater amount of fluid may be necessary to constrict the portion of the patient's stomach. If the patient is mildly obese, then a lesser degree of restriction, and less fluid may be passed to the inflatable member **24**, than if the patient is severely obese.

The patient's stomach is constricted in order to treat obesity. The constriction causes the food to pass from the patient's esophagus **28** to the lower portions of the patient's stomach **22**, at a rate that is slower than would normally occur without the restriction. The decreased rate of food flow increases a feeling of fullness for the patient, and enhances satiety signals that are sent to the patient's brain. The enhanced feeling of fullness causes the patient to reduce food consumption, which desirably causes the patient to lose weight.

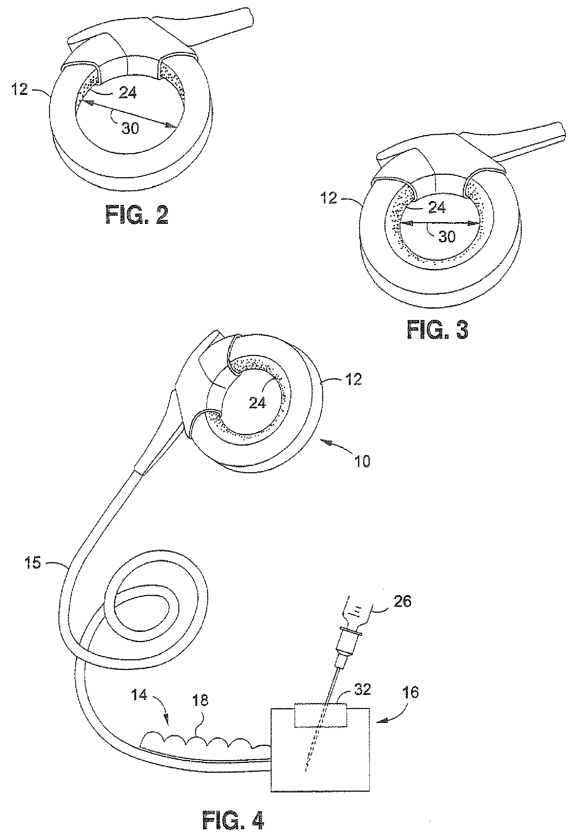

FIG. 2 illustrates a perspective view of the gastric band **12** and the inflatable member **24** of FIG. 1. The gastric band **12** is designed to form a loop around the patient's stomach, and form an inner diameter **30** which defines the size of a constriction of the patient's stomach. If the inner diameter **30** size is reduced, then the restriction of the patient's stomach increases. The size of the constriction, or constricted portion of the patient's stomach, accordingly decreases.

FIG. 3 illustrates a perspective view of the gastric band **12** with an increased amount of fluid passed into the inflatable member **24**. The diameter **30** size is reduced, causing an increased restriction of the patient's stomach, and accordingly, a decreased size of the constriction.

FIG. 4 illustrates a perspective view of the gastric banding system **10** of FIG. 1, including the gastric band **12** and the adjustment system **14**. The syringe **26** is illustrated to penetrate a septum **32** of the access port **16**, in a position that would allow a physician to vary an amount of fluid in the inflatable member **24**.

FIG. 5A illustrates a schematic representation of the constriction of a portion of the patient's stomach formed by the gastric band **12**. The inflatable member **24** encircles a portion of the patient's stomach to form a constriction. The diameter **30** of the gastric band defines a size of a gastric lumen **33**, or interior cavity of the patient's stomach, for food to pass through, from the patient's esophagus to the lower portions of the patient's stomach. The size, or cross-sectional surface area, of the lumen **33** defines the size of the passageway through which food can pass. If the lumen **33** is small, then food can not easily pass through the lumen **33.** If the lumen **33** is large, then food can more easily pass through the lumen **33**.

In a standard gastric banding system, the size of the constriction of the patient's stomach, and the size of the gastric lumen **33**, is fixed. In other words, in a standard gastric banding system, after the gastric band and the access port have been implanted into a patient's body, the size of the lumen **33** is set by the physician, via the access port. In one embodiment, the size of the lumen **33** may only be adjusted if the physician inserts a syringe into the access port and adds or removes fluid from the gastric band lumen **33**. However, a lumen **33** with a fixed size may be undesireable if an obstruction, or a large bolus of food, attempts to pass through the lumen **33**. The obstruction may become stuck in the lumen **33** as it may not be able to pass through the construction formed by the gastric band. This result is undesirable because it could cause pain for the patient. The patient may need to visit a physician to loosen the band in order to allow the obstruction to pass through the constriction. The obstruction may comprise a large bolus of food traveling through the esophagus to the patient's stomach. The obstruction may also comprise the patient's vomit, attempting to travel from the patient's stomach up through the patient's esophagus.

It is thus desirable if an adjustment system **14** is incorporated into the gastric banding system **10**, to allow a size of a portion of the gastric lumen **33** constricted by a gastric band to vary in response to an obstruction passing through the gastric lumen **33**.

The components and operation of the adjustment system **14** will now be discussed in relation to FIGS. 5A and 5B. As shown in FIG. 5A, and as discussed in relation to FIG. 1, the adjustment system **14** includes an access port **16**, a reservoir **18**, and an asymmetric flow regulator **20** (visible in FIG. 5B).

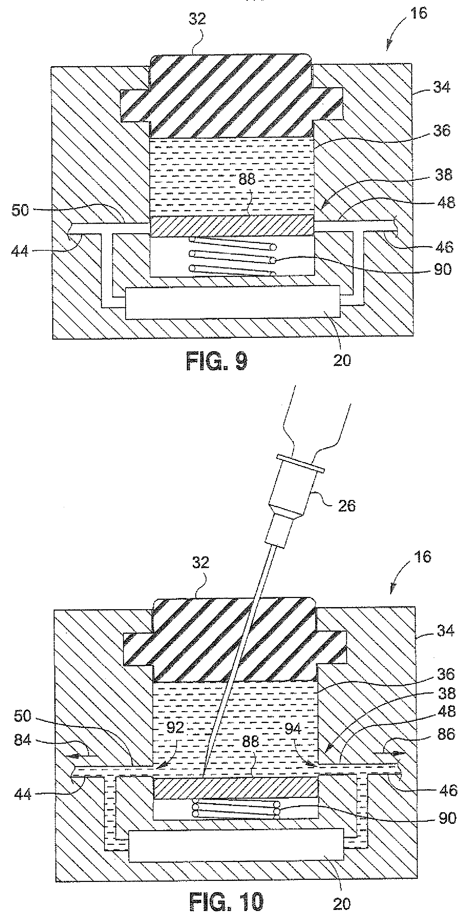

The access port **16** includes a housing **34**, a septum **32** and a fluid chamber **36** (visible in FIGS. 9 and 10). The housing **34** forms an outer shell of the access port **16**, and retains the septum **32** and the fluid chamber **36**. The housing **34** is configured to be biocompatible, to allow the housing **34** to integrate biologically with the local tissues positioned around the housing **34**. The septum **32** is configured to allow a syringe needle to pass through the septum **32** and enter the fluid chamber **36**, to add or remove fluid from the gastric band **12**. The septum 32 prevents fluid from exiting the fluid chamber **36** when the syringe needle penetrates through the septum **32**, and after the syringe needle is withdrawn from the septum **32**.

The reservoir **18** comprises a structure configured to receive fluid from the gastric band **12.** The reservoir **18** may comprise a flexible, or elastic, structure having a volume capable of automatically varying to accommodate an amount of fluid entering the reservoir **18**. In the embodiment shown in FIG. 5A, the reservoir **18** may comprise a flexible balloon or bladder structure, capable of increasing in size in response to an amount of fluid entering the reservoir **18**. The reservoir **18** may stretch to accommodate fluid entering the reservoir **18**, and may shrink to accommodate fluid exiting the reservoir **18**. In addition, as shown in FIG. 5A, the reservoir **18** may include a ridged structure **17**, including a plurality of notches on the surface of the reservoir **18**. The notches enhance the flexibility of the reservoir **18** by forming pre-stressed regions of the reservoir **18**.

The physical properties of the reservoir **18** may be selected as desired to effect various performance attributes of the adjustment system **14**. For example, a relatively large reservoir **18** may allow the reservoir **18** to more quickly receive fluid from the gastric band **12**. A larger reservoir **18** will allow a large quantity of fluid to pass from the gastric band **12** to the reservoir **18**. In addition, a more flexible reservoir **18** may allow fluid to more quickly pass to the reservoir **18** from the gastric band **12**. Further, a smaller reservoir **18** may reduce the total size of the adjustment system **14**. Moreover, a less flexible reservoir **18** may reduce the disruption caused by the reservoir **18** towards local tissues, when the reservoir **18** inflates with fluid. The reservoir **18** may be made of an elastic resilient material, such as silicone, or may be made of a rubber or appropriate plastic. The materials selected to form the reservoir **18** may be varied as desired, and the size of the reservoir **18** may be varied as desired. In one embodiment, the reservoir **18** may be sized such that the gastric banding system **10** contains approximately 56 mL (fifty-six milliliters) of fluid. This volume is exemplary and may be varied as desired.

The reservoir **18** is coupled to the housing **34** through a coupling device, namely, through a tube coupler or a connecting nipple structure. Other appropriate coupling devices may be utilized as desired. The reservoir **18** may be securely or detachably fixed to the housing **34.** In an embodiment in which the reservoir **18** is detachably fixed to the housing **34**, a detachable locking mechanism may be used to couple the reservoir **18** to the housing **34**.

The reservoir **18** is preferably positioned exterior to the housing **34** to accommodate size changes of the reservoir **18**. In an embodiment in which the reservoir **18** comprises a bladder or balloon configured to vary in size, the externally placed reservoir **18** may allow the reservoir **18** to expand without being confined by the size of the housing **34**. In one embodiment, the reservoir **18** may be positioned external to the housing **34**, yet placed within a shell that is configured to house the reservoir 18. The shell may be sized to allow the reservoir **18** to increase or decrease in size without interference from the interior surfaces of the shell (e.g., restrained by the interior surfaces of the shell).

Further, the reservoir **18** is preferably positioned exterior to the housing **34**, to allow the reservoir **18** to be removed from the housing **34** and replaced as necessary. As discussed above, the physical properties of the reservoir **18** may be varied as desired. A physician may determine a larger reservoir **18** is necessary for use in the adjustment system **14**. An external reservoir may more easily allow a physician to access the reservoir **18** and replace the reservoir **18**, without having to remove the gastric band **12** or the access port **16** from the patient's body.

FIG. 5A illustrates the reservoir **18** extending along the length of the tube **15**, and extending in a direction towards the gastric band **12**. The position of the reservoir **18** along the tube **15** allows the profile of the adjustment system **14** to be reduced, as the tissue displaced by the reservoir **18** has already been displaced by the path of the tube **15** leading towards the gastric band **12**. Further, FIG. 5A illustrates the reservoir **18** positioned above the tube **15**. The position of the reservoir **18** above the tube **15** allows the physician to more easily access the reservoir **18** without interference from the tube **15** extending from the housing **34**.

FIG. 5B illustrates a schematic view of the gastric banding system **10** shown in FIG. 5A. The gastric banding system **10** includes the gastric band **12**, schematically outlined in a dashed box, and the adjustment system **14**. The adjustment system **14** includes the reservoir **18**, schematically outlined in a solid box, and the asymmetric flow regulator **20**, schematically outlined in a dashed box. The access port housing **34** is schematically represented by a dashed box. The asymmetric flow regulator **20** and a shunt valve **38** are shown positioned within the access port housing **34**. A schematic representation of a filling point **40** is also shown, positioned within the access port housing **34**.

The tube **15** connects the gastric band **12** to the access port housing **34**. A fluid conduit **44** connects the tube **15** to the asymmetric flow regulator **20**. A coupler **42** connects the reservoir **18** to the access port housing **34**. A fluid conduit **46** connects the asymmetric flow regulator **20** to the coupler **42**. A fluid conduit **48** connects the shunt valve **38** to the conduit **46** leading to the coupler **42.** A fluid conduit **50** connects the shunt valve **38** to the conduit **44** leading to the tube **15.**

The asymmetric flow regulator **20** comprises a mechanism including a valve that is configured to open to allow fluid from the gastric band **12** to pass through the valve, when a pressure of incident fluid exceeds a threshold. The asymmetric flow regulator **20** further comprises a flow restriction device that is configured to allow fluid from the reservoir **18** to pass to the gastric band **12**. In the embodiment shown in FIG. 5B, the valve is configured as a check valve, or a one-way valve **52**, that only allows flow in one direction, and opens when an incident fluid pressure exceeds a threshold pressure.

In the embodiment shown in FIG. 5B, the one-way valve **52** comprises a variable resistance ball check valve, or a check valve that prevents fluid flow in a first direction by the force of a spring pressing against a ball. The one-way valve **52** is moveable from a closed position, in which no flow is allowed, to an open position, in which flow is allowed through the one-way valve **52**. The one-way valve **52** only allows flow in the opposite or second direction, to the first direction, when a pressure of incident fluid exceeds a defined threshold value. The one-way valve **52** thus only allows flow in the direction of: from the inflatable member **24** to the reservoir **18**. The one-way valve **52** opens automatically when the pressure of incident fluid exceeds a defined threshold value. The threshold value is defined by the degree of force the spring exerts against the ball.

The threshold value of the one-way valve **52** can be adjusted by varying the compressive force of the spring against the ball, as desired. The compressive force may be varied either mechanically, or hydraulically, as is known in the art. For example, a twisting mechanism may vary the spring constant of the spring, and consequently increase the compressive force of the spring. In addition, a hydraulic pressure may press against the spring to vary the compressive force of the spring. The threshold value of the one-way valve **52** may be adjusted either prior to implantation of the housing **34** or after the housing **34** has been implanted into the patient's body. In one embodiment, the physician may insert a syringe into the housing **34**, after the housing **34** has been implanted, to hydraulically vary the threshold value of the one-way valve **52**. The threshold value is defined by a structural configuration of the one-way valve **52**, or, in other words, a physical property of the one-way valve **52** (e.g., the tension of the spring) defines the value of the threshold force required to open the valve **52**.

In one embodiment, the one-way valve **52** may be configured as a valve such as an adjustable diaphragm valve, or adjustable duckbill valve. In one embodiment, the one-way valve **52** may be adjusted through telemetric means, in which a physician wirelessly communicates with a controller that controls the opening and closing of the one-way valve **52**, and the threshold opening pressure of the one-way valve **52**. In one embodiment, the one-way valve **52** may be configured to comprise any equivalent mechanism capable of only allowing fluid flow in one direction. In one embodiment, the valve of the asymmetric flow regulator **20**, shown in FIG. 5B, to comprise a one-way valve **52**, may comprise any mechanism capable of opening to allow fluid flow when incident fluid pressure is above a threshold pressure value.

In the embodiment shown in FIG. 5B, the threshold value at which the one-way valve **52** may allow fluid flow, may be set as a pressure differential across the one-way valve **52**. In other words, the one-way valve **52** may open when the pressure of fluid incident from the inflatable member **24** of the gastric band **12** is greater than the pressure of fluid contained within the reservoir **18**. For example, a pressure differential of 10mmHg (ten millimeters of mercury) across the one-way valve **52** may cause the one-way valve **52** to open. This threshold value is exemplary, and may be varied as desired. In one embodiment, the threshold value may be an absolute value, of which the one-way valve **52** opens when the pressure of fluid incident from the inflatable member **24** raises above this value. The absolute pressure value may be, for example, 20mmHg. The absolute pressure value may also be in a range of between approximately 10mmHg to 80mmHg. This threshold value is exemplary, and may be varied as desired.

The asymmetric flow regulator **20** preferably includes a flow restriction device, shown in FIG. 5B, to comprise a flow control valve **54**. The flow control valve **54** preferably comprises a device that reduces the flow rate of fluid passing through the flow control valve **54**. The flow control valve **54** may comprise a narrow fluid conduit that includes an obstruction in the flow path of the conduit. The flow rate through the flow control valve **54** may be reduced by narrowing the flow path for fluid passing through the flow control valve **54**, by reducing the cross-sectional area for the fluid to pass through by placing the obstruction in the flow path, to impede the passage of the fluid through the flow control valve **54**. In one embodiment, the flow rate may be defined as a volume of fluid passing through the flow control valve **54** per unit time.

In one embodiment, the flow control valve **54** may comprise a variable flow control valve **54**, capable of varying the flow rate passing through the flow control valve **54**. For example, the flow control valve **54** may comprise a tube having inflatable walls that surround a fluid passageway. The rate of fluid passage through the walls depends on the degree to which the walls are inflated. The flow rate may therefore be varied by varying the amount of fluid contained within the walls of the valve **54**, and therefore varying the size of the walls. The amount of fluid in the walls may be varied prior to, or after implantation of the housing **34.** A physician may insert a syringe into the access port housing **34** to add or remove fluid from the walls of the valve **54.**

In one embodiment, the flow restriction device may comprise a narrow fluid conduit, being sized more narrowly than other fluid conduits utilized in the adjustment system **14**, for example, conduits **44**, **46**, **48**, **50**, or fluid conduits **56**, **58**, **60**, **62**. For example, in this embodiment, the flow restriction device may comprise a narrow tubing. This tubing may allow fluid to pass from the reservoir **18** to the gastric band **12** at a rate that is less than a rate at which fluid passes through the one-way valve **52**.

In one embodiment, the flow restriction device may comprise any mechanism capable of reducing the flow rate of fluid passing therethrough. In one embodiment, the restricted flow rate through a flow restriction device may be defined as a rate, being less than the rate at which fluid is capable of flowing through the one-way valve **52**, when the one-way valve **52** is open. In one embodiment, the restricted flow rate through a flow restriction device may be defined as a rate that causes the inflatable member **24** of the gastric band **12** to fill at a rate, which is less than the rate at which fluid exits the gastric band **12**, when it passes through the one-way valve **52**.

The various conduits **44**, **46**, **48**, **50**, **56**, **58**, **60**, **62** shown schematically in FIG. 5B may be implemented as tubes, channels, or passageways. In one embodiment, the conduits **44**, **46**, **48**, **50**, **56**, **58**, **60**, **62** may be formed as structural elements of the access port housing **34**. In one embodiment, the conduits **44**, **46**, **48**, **50**, **56**, **58**, **60**, **62** may be formed during the molding process that is used to form the access port housing **34**.

In the embodiment shown in FIG. 5B, a fluid conduit **56** fluidly links the one-way valve **52** with the conduit **44** that fluidly communicates with the inflatable member **24**. A fluid conduit **58** also fluidly links the one-way valve **52** with the conduit **46** that fluidly communicates with the reservoir **18.** The fluid conduits **56**, **58** also link the one-way valve **52** with the fluid conduits **60**, **62** leading to the flow control valve **54.** The one-way valve **52** is therefore in fluid communication with the inflatable member **24** of the gastric band **12**, and with the flow control valve **54,** and with the reservoir **18.** The one-way valve **52** is also in fluid communication with the shunt valve **38**.

In the embodiment shown in FIG. 5B, a fluid conduit **60** fluidly links the flow control valve **54** with the conduit **44** that fluidly communicates with the gastric band **12.** A fluid conduit **62** fluidly links the flow control valve **54** with the conduit **46**, which fluidly communicates with the reservoir **18.** The fluid conduits **60, 62** also link the flow control valve **54** with the fluid conduits **56, 58** leading to the one-way valve **52.** The flow control valve **54** is therefore in fluid communication with the inflatable member **24** of the gastric band **12,** with the one-way valve **52,** and with the reservoir **18.** The flow control valve **54** is also in fluid communication with the shunt valve **38**.

In the embodiment shown in FIG. 5B, the one-way valve **52** and the flow control valve **54** are in fluid communication with the inflatable member **24** of the gastric band **12** in a parallel configuration. In addition, the one-way valve **52** and the flow control valve **54** are in fluid communication with the reservoir **18** in a parallel configuration. The fluid may flow from the inflatable member **24** of the gastric band **12** through either the flow control valve **54,** or through both the flow control valve **54** and the one-way valve **52,** depending on the pressure of the fluid passing from the inflatable member **24**. The one-way valve **52** is in direct fluid communication with the reservoir **18** and the inflatable member **24**, because no valve or control device impedes fluid flow between the one-way valve **52** and the reservoir **1**8**,** and the one-way valve **52** and the inflatable member **24**. The one-way valve **52** is also in direct fluid communication with the flow control valve **54**, because no valve or control device impedes fluid flow between the one-way valve **52** and the flow control valve **54.** The flow control valve **54** is in direct fluid communication with the reservoir **18** and the inflatable member **24**, because no valve or control device impedes fluid flow between the flow control valve **54** and the reservoir **18,** and the flow control valve **54** and the inflatable member **24.**

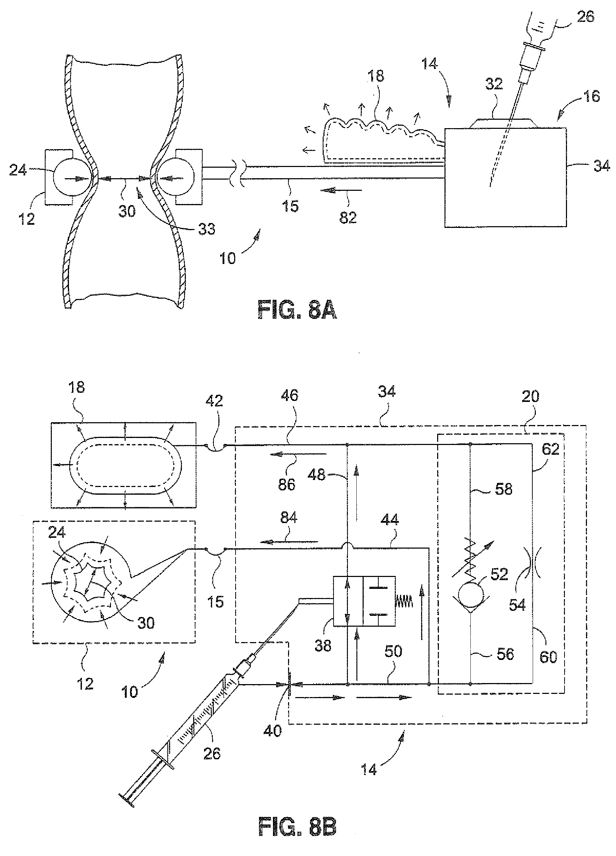

The shunt valve **38,** to be discussed further in relation to FIGS. 9A and 9B, comprises a valve that is opened or closed at the selection of a user, who preferably comprises a physician. In the configurations of the gastric banding system **10** shown through FIGS. 5A through 7B, the shunt valve **38** remains closed, to prevent fluid from passing through the shunt valve **38**. The shunt valve **38** is shown in an open configuration in FIGS. 8A and 8B.

Referring back to FIG. 5A, in operation, the adjustment system **14** automatically increases the size of the constricted portion of the gastric lumen **33** in response to an obstruction passing through the gastric lumen **33**. The size of the constricted portion of the gastric lumen **33** increases rapidly when the obstruction approaches the gastric band **12**. The fluid that fills the inflatable member **24** rapidly travels through the tube **15** and fills the reservoir **18**. The decreased amount of fluid in the inflatable member **24** increases the diameter **30** of the gastric band **12,** and increases a size of the gastric lumen **33.**

The adjustment system **14** is also configured to slowly return the size of the constricted gastric lumen **33** to the size it had prior to the obstruction passing through the lumen **33,** or the size when no obstruction is passing through.

FIGS. 5A and 5B illustrate the gastric banding system **10** in an equilibrium state, in which the gastric band **12** is in an equilibrium position. The equilibrium position may be equivalently referred to as the constricted state of the inflatable member **24**. In the equilibrium state, the pressure of fluid within the inflatable member **24** is equal, or nearly equal to the pressure of fluid within the reservoir **18.** The pressure of the inflatable member **24,** or the pressure difference between the inflatable member **24** and the reservoir **18,** is not sufficient to open the one-way valve **52**. In this state, fluid may only pass from the inflatable member **24** to the reservoir **18** through the flow control valve **54**.

The equilibrium pressure of the inflatable member **24** and the reservoir **18** is preferably set by a physician, to optimally reduce food intake for the patient, depending on the unique physical characteristics and size of the patient. For example, if the physician desires that a greater degree of constriction be applied to the gastric lumen **33,** then a greater amount of fluid may be passed into the gastric banding system **10,** which increases the equilibrium pressure. A physician may adjust the equilibrium pressure by varying the amount of fluid in the gastric banding system **10** through use of the shunt valve **38**, in a process discussed more fully in relation to FIGS. 8A and 8B.

Forces may be exerted against the gastric band **12** that cause the equilibrium state to be disturbed. If the equilibrium state is disrupted by forces, to the extent that the one-way valve **52** does not open, then fluid will slowly pass through the flow control valve **54** between the reservoir **18** and the inflatable member **24,** to maintain the equilibrium state, between the pressures of the inflatable member **24** and the reservoir **18.** For example, the one-way valve's **52** threshold pressure may be set at a differential of 10mmHg between the inflatable member **24** and the reservoir **18**. Further, the pressure of the inflatable member **24** in an equilibrium state may be 11mmHg, and the pressure of the reservoir **18** may be 11mmHg. If forces are exerted against the gastric band **12** to raise the pressure of the inflatable member **24** to 15mmHg, then the 10mmHg differential has not been met. Fluid will then only flow through the flow control valve **54** at a slow flow rate, until the pressures of the inflatable member **24** and the reservoir **18** equalize at 15mmHg. The size of the gastric lumen **33** and the size of the gastric band's **12** diameter **30** increase in response to the fluid being transferred from the inflatable member **24** to the reservoir **18**.

The slow flow of fluid through the flow control device **54** beneficially maintains equilibrium across the inflatable member **24** and the reservoir **18**, and adjusts a size of the gastric lumen **33** in response to prolonged forces exerted against the gastric band **12**. The flow through the flow control device **54**, and the adjusted size of the gastric lumen **33** occur until an equilibrium pressure is reached between the pressures of the inflatable member **24** and the reservoir **18.**

The slow flow of fluid through the flow control valve **54**, however, may not be sufficient to adjust a size of the gastric band **12** quickly, in response to an obstruction passing through the gastric lumen **33**. An alternate mechanism, namely, the one-way valve **52,** is utilized to allow fluid to pass at a large, or fast, flow rate from the inflatable member **24** to the reservoir **18,** to quickly increase the size of the gastric lumen **33**, and accordingly quickly increase the diameter **30** of the gastric band **12.** The fast flow rate may be defined as a rate being faster than fluid may pass through the flow restriction device, represented in FIG. 5B as a flow control valve **54**.

A large force exerted against the gastric band **12** will overcome the threshold pressure of the one-way valve **52.** The one-way valve **52** will open in response to pressure of the fluid in the inflatable member **24** increasing over a threshold. The large force exerted against the gastric band **12** may be caused by an obstruction passing through the gastric lumen **33**. Fluid will pass from the inflatable member **24** to the reservoir **18** at a fast flow rate through the one-way valve **52**. Fluid will pass through the one-way valve **52** until the threshold pressure differential is met, at which time the one-way valve **52** will close. The remaining pressure differential may be equalized through fluid flow through the flow control valve **54.**

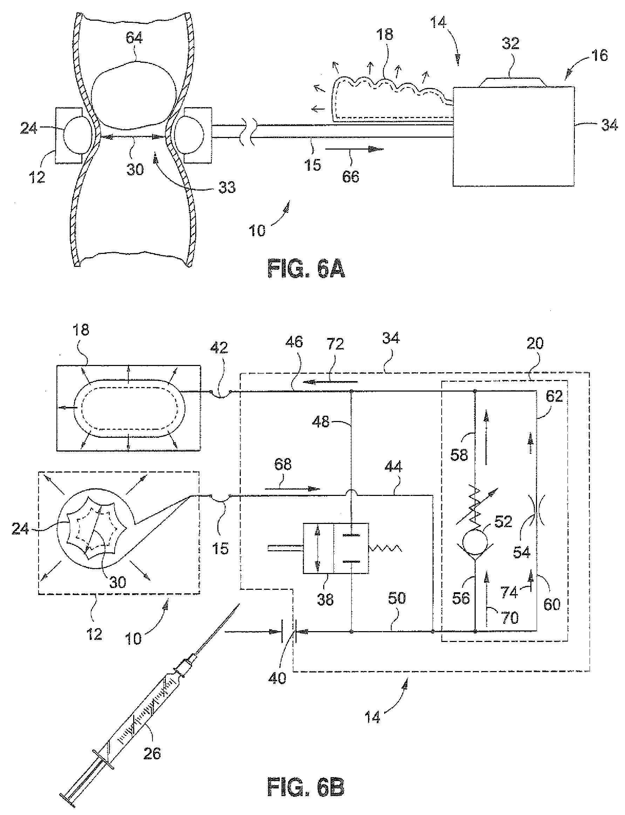

FIG. 6A illustrates a schematic representation of an obstruction **64** passing through the patient's gastric lumen **33**. The obstruction **64** represents any of the embodiments of an obstruction discussed throughout this disclosure, including a large bolus of food. The obstruction **64** may also comprise any other object, which could not pass through the constriction formed by the gastric band **12,** when the gastric band **12** is at its equilibrium position. In the embodiment shown in FIG. 6A and 6B, if the obstruction **64** exerts a force against the gastric band **12** large enough to overcome the pressure threshold of the one-way valve **52,** then the one-way valve **52** will open, and will allow fluid to pass from the inflatable member **24** to the reservoir **18** at a fast flow rate. FIG. 6A illustrates such a state of the gastric banding system **10,** in which the force from the obstruction **64** against the gastric band **12** is sufficient to overcome the pressure threshold of the one-way valve **52.**

As shown in FIG. 6A, a long arrow **66** representing fluid flow at a fast rate, indicates fluid passing from the inflatable member **24** to the reservoir **18.** The size of the diameter **30** of the gastric band **12** increases, as the volume of fluid in the inflatable member **24** is reduced. The size of the reservoir **18** increases, as the fluid has passed from the inflatable member **24** to the reservoir **18.**

FIG. 6B illustrates a schematic view of the gastric banding system **10** in the state shown in FIG. 6A. The diameter of the inflatable member **24** of the gastric band **12** increases. The inflatable member **24** moves to a passage state, or passage size, for allowing the obstruction **64** to pass through the gastric lumen **33**. Less fluid is contained in the inflatable member **24** in the passage state than in the constricted state. The inflatable member **24** is moveable from the passage state, to the constricted state, and from the constricted state to the passage state.

A long arrow **68** indicates a fluid flow at a fast flow rate passing to the one-way valve **52**. A long arrow **70** indicates fluid flow at a fast flow rate passing through the one-way valve **52**. A long arrow **72** indicates a fluid flow at a fast flow rate passing to the reservoir **18**. A short arrow **74** indicates a fluid flow at a slow flow rate through the flow control valve **54.** The fast flow rate is greater than the slow flow rate.

FIG. 6B further illustrates the size of the reservoir **18** increasing as it fills with fluid. The reservoir **18** receives the fluid that is passed through the one-way valve **52,** to allow a size of the portion of the gastric lumen **33** being constricted, to increase in response to the obstruction passing through the gastric lumen **33,** to allow the obstruction to pass through the portion of the gastric lumen **33.**

The adjustment system **14** beneficially rapidly increases the size of the portion of the gastric lumen **33** that is constricted in response to an obstruction **64** passing through the gastric lumen **33**. The adjustment system **14** does so automatically, or without user intervention, in response to luminal **33** pressure increasing or rising above a threshold level. The rapid increase of the size of the portion of the gastric lumen **33** beneficially allows the obstruction **64** to quickly pass through the constriction of the gastric lumen **33**. It is desired that the diameter **30** of the inflatable member **24** is able to increase to the passage state, or passage size, which allows the obstruction **64** to pass through the gastric lumen **33.**

The flow of fluid from the inflatable member **24** to the reservoir **18** occurs until the pressure of the reservoir **18** equals or is approximately equal to the pressure of the inflatable member **24.**

The force exerted by the obstruction **64** against the gastric band **12** will be reduced once the obstruction **64** passes through the constriction of the gastric lumen **33.** The fluid pressure in the reservoir **18** will then exceed the pressure in the inflatable member **24** of the gastric band **12.** The pressure differential will cause fluid to flow back from the reservoir **18** to the inflatable member **24** of the gastric band **12**, until the pressure between the inflatable member **24** and the reservoir **18** is again equalized. The fluid returning from the reservoir **18** to the inflatable member **24** can not pass through the one-way valve **52**. Instead, the fluid will return to the inflatable member **24** through the flow control valve **54,** at a slow flow rate.

In one embodiment, the reservoir **18** may be configured to exert a compressive, or elastic, force against the fluid contained within the reservoir **18.** The force may further contribute to the pressure of the reservoir **18**.

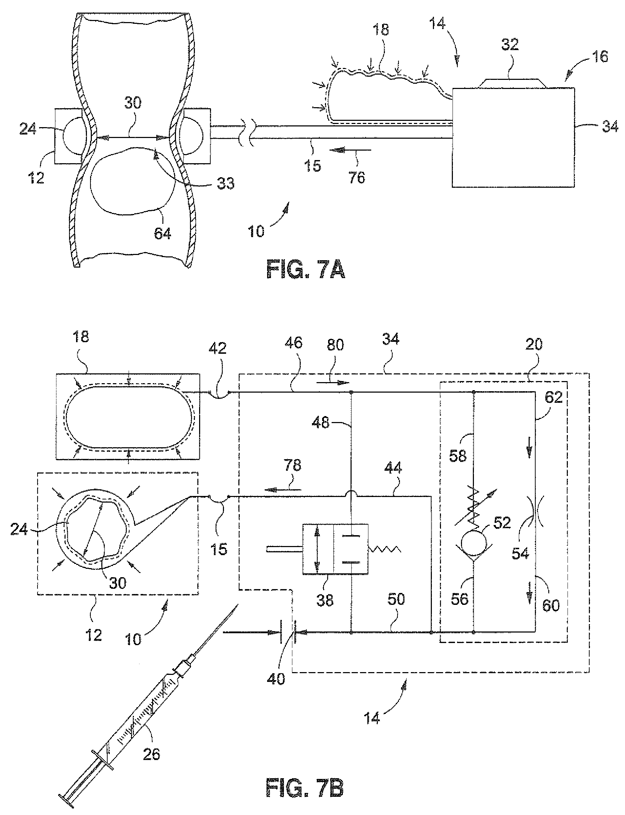

FIG. 7A illustrates a schematic representation of the state of the gastric banding system **10** after the obstruction **64** has passed through the portion of the patient's gastric lumen **33** being constricted. A short arrow **76** indicates fluid flow at a slow rate from the reservoir **18** to the inflatable member **24** of the gastric band **12**. The reservoir **18** slowly decreases in size due to fluid exiting the reservoir **18** at a slow rate.

FIG. 7B illustrates a schematic view of the gastric banding system **10** in the state shown in FIG. 7A. The diameter **30** of the inflatable member **24** of the gastric band **12** is shown to slowly decrease. The inflatable member **24** slowly returns to the constricted state shown in FIGS. 5A and 5B. A short arrow **78** indicates fluid flowing at a slow rate from the flow control valve **54** to the inflatable member **24** of the gastric band **12**. A short arrow **80** indicates fluid flowing at a slow rate from the reservoir **18** to the flow control valve **54.**

The slow flow rate of fluid from the flow control valve **54** to the inflatable member **24** beneficially constricts the gastric lumen **33** slowly after the obstruction **64** has passed. The slow return reduces the possibility of damage to the patient's stomach that could be caused by a relatively quick return of fluid to the inflatable member **24**. The slow return of fluid allows the patient's stomach to be slowly compressed, which reduces the possibility of local tissues rupturing or becoming damaged. In addition, wear on the gastric banding system **10** is reduced. Furthermore, the slow return maintains the diameter **30** of the gastric band **12** at an increased size, or the passage state, for a prolonged period of time. It is beneficial to maintain the diameter **30** of the gastric band **12** in the passage state, because it is expected that if one obstruction **64** has passed through the constriction of the gastric lumen **33**, then it is likely that another obstruction **64** may be forthcoming. For example, if the patient is eating many large pieces of steak, then it is likely that after one large piece of steak is consumed, then another will be subsequently consumed. The slow return of fluid may keep the gastric band **12** open for a duration that allows for a successive obstruction **64** to more easily pass through the constriction, before the construction closes again. The gastric band **12** will not have to open and close quickly for each successive obstruction **64** passing through the patient's gastric lumen **33**.

In one embodiment, the time for the gastric band **12** to open and return to the equilibrium state may be approximately 15 minutes. In one embodiment, the time for the gastric band **12** to open and return to the equilibrium state may be in a range of between approximately 1 minute and 15 minutes. These durations are exemplary in nature and may be varied as desired.

FIG. 8A illustrates a schematic representation of a state of the gastric banding system **10** in which a user, preferably a physician, adjusts the volume of fluid in the inflatable member **24** of the gastric band **12** and the reservoir **18**. The adjusted volume of fluid will also adjust the equilibrium pressure of the gastric band **12** and the reservoir **18**, as was discussed in relation to FIGS. 5A and 5B.

In the embodiment shown in FIG. 8A, the physician adjusts the volume of fluid in the gastric banding system **10** by inserting a syringe **26** into the access port housing **34**, and either adding or removing fluid from the system. FIG. 8A shows fluid being added to the gastric banding system **10**. The long arrow **82** indicates fluid flow to the inflatable member **24**. The diameter **30** of the gastric band **12** decreases as the size of the inflatable member **24** increases. The size of the reservoir **18** increases.

FIG. 8B illustrates a schematic view of the gastric banding system **10** in the state shown in FIG. 8A. The operation of adjusting the volume of fluid in the gastric banding system **10** is shown to be accomplished through the opening of the shunt valve **38.** The shunt valve **38** may comprise a valve capable of being switched between an open position and a closed position. The shunt valve **38** is preferably held in a closed position through a plate and a spring mechanism, which is opened when a force is exerted against the plate by the syringe **26.** The spring biases the plate to return the shunt valve **38** to the closed position once the syringe **26** is removed. In the embodiment shown in FIG. 8B, the shunt valve **38** is shown to comprise a valve that may be switched between an open position and a closed position, through a force exerted by the passage of the syringe **26** into the housing **34**.

As discussed in relation to FIG. 5B, the shunt valve **38** is directly connected to the reservoir **18** through a fluid conduit **48**, and is directly connected to the inflatable member **24** of the gastric band **12** through a fluid conduit **50.** The shunt valve **38** is configured to allow fluid to pass to the reservoir **18** and the inflatable member **24** without having to pass through the asymmetric flow regulator **20.** The shunt valve **38** is in direct fluid communication with the reservoir **18** and the inflatable member **24**, as no valve or control device impedes fluid flow between the shunt valve **38** and the reservoir **18**, and the shunt valve **38** and the inflatable member **24**. The shunt valve **38** is also in direct fluid communication with the one-way valve **52** and the flow control valve **54.**

In one embodiment, the shunt valve **38** may comprise a valve capable of being opened by a fluid pressure exerted against the valve **38,** by fluid exiting or entering the syringe **26.** In one embodiment, the shunt valve **38** may comprise any valve capable of producing equivalent operation, capable of opening and closing to allow fluid to flow to the reservoir **18** and the inflatable member **24,** without having to pass through the asymmetric flow regulator **20.**

The operation of the shunt valve **38** is schematically represented in FIG. 8B as the valve element, indicated by the reference number **38,** and a filling point **40.** The valve element indicated by reference number **38** represents the valve feature of the shunt valve **38,** and the filling point **40** represents the ability of the shunt valve **38** to receive and withdraw fluid from the syringe **26.**

FIG. 8B illustrates a state of the system **10** in which the shunt valve **38** is open, and fluid is able to flow to the inflatable member **24** and the reservoir **18.** A long arrow **84** represents a rapid flow of fluid, or fast flow rate, to the inflatable member **24** through the fluid conduit **44**. A long arrow **86** represents a fast flow rate to the reservoir **18** through the fluid conduit **46.** The inflatable member **24** inflates with fluid, reducing the diameter of the gastric band **12.** Further, the reservoir **18** inflates with fluid. The fast flow rate may be defined as a rate being faster than fluid may pass through the flow restriction device, which is shown in FIG. 8B to comprise a flow control valve **54.**

FIG. 9 illustrates a cross-sectional view of one embodiment of the access port **16,** illustrating the shunt valve **38.** The access port **16** includes a housing **34,** a septum **32,** and a fluid chamber **36** contained within the housing **34.** The septum **32** covers the fluid chamber **36,** to prevent fluid from leaving the chamber **36**. The septum **32** is preferably needle penetrable and self sealing, and is made from a material such as silicone.

The shunt valve **38** is integrated with the fluid chamber **36**. The shunt valve **38** includes a plate **88** and a spring **90** that biases the plate **88** in a direction towards the septum **32**. The plate **88** is biased to block passage of fluid from the fluid conduit **50** leading to the inflatable member **24** (shown in FIG. 8B), to the fluid conduit **48** leading to the reservoir **18** (shown in FIG. 8B). The closed fluid passage between the inflatable member **24** and the reservoir **18** allows fluid to only travel through the asymmetric flow regulator **20.**

FIG. 10 is a cross-sectional view of the access port **16** shown in FIG. 9, after a syringe **26** needle has penetrated the septum **32** and contacted the plate **88.** FIG. 10 also represents the state of the access port **16** shown schematically in FIG. 8B. The force of the syringe **26** needle against the plate **88** causes the spring **90** to compress, and opens up a fluid channel **94** leading to the reservoir **18**, and opens up a fluid channel **92** leading to the inflatable member **24**. Once the valve **38** opens, the fluid within the fluid chamber **36** equalizes pressure with the fluid contained in the reservoir **18** and the inflatable member **24.** Fluid may then pass from the syringe **26** to and from the reservoir **18** and the inflatable member **24,** through the respective channels **94**, **92**. The long arrows **84** and **86** represent fluid flow to the respective inflatable member **24** and the reservoir **18.** Fluid does not need to pass through the asymmetric flow regulator **20** to reach the reservoir **18** or the inflatable member **24.**

After the syringe **26** needle is removed, the shunt valve **38** closes, and the access port **16** returns to the state shown in FIG. 9.

The shunt valve **38** beneficially allows a physician to adjust the fluid level in the reservoir **18** and inflatable member **24**, without fluid having to pass through the asymmetric flow regulator **20**. For the embodiment of the asymmetric flow regulator **20** shown in FIG. 8B, if fluid passed through the asymmetric flow regulator **20,** then it would either need to pass through the one-way valve **52,** or the flow control valve **54.**

For example, if fluid was entered into the system on the side of the one-way valve **52** that is coupled to the reservoir **18**, then the fluid could not pass to the inflatable member **24** through the one-way valve **52.** Fluid may only flow through the one-way valve **52** in a direction of: from the gastric band **12** to the reservoir **18.** The fluid could only pass slowly through the flow control valve **54.** In addition, if fluid was entered into the system on the side of the one-way valve **52** that is coupled to the inflatable member **24,** then it could only pass through the one-way valve **52** if the fluid pressure exceeded the threshold pressure of the one-way valve **52.** The fluid could otherwise only pass at a slow rate through the flow control valve **54.** Thus, without the shunt valve **38,** there would be a delayed response between the insertion or removal of fluid through the syringe **26**, and the insertion or removal of fluid from the reservoir **18** and/or the inflatable member **24,** caused by fluid passing slowly through the flow control valve **54.**

The shunt valve **38** also beneficially allows the physician to quickly ascertain the fluid pressures of the reservoir **18** and the inflatable member **24.** If the physician uses a syringe **26** having an integrated pressure meter, then the physician may receive more current readings of the fluid pressure in the system **12,** than if fluid had to pass through the flow control valve **54** at a slow rate. The shunt valve **38** may lead to more accurate and rapid adjustments of the fluid volumes and pressures in the gastric banding system **10**.

The shunt valve **38** also beneficially allows the gastric band **12** to be emptied rapidly without delay. If the gastric band **12** must be quickly removed from the patient's body, the shunt valve **38** may allow the fluid in the gastric band **12** to quickly be extracted if necessary.

The cross sectional view of the access port **16** shown in FIGS. 9 and 10 represent an exemplary configuration of the access port **16**. In other embodiments, the asymmetric flow regulator **20** may be positioned in alternative locations throughout the access port housing **34,** which produce equivalent results. In addition, the fluid conduits **44**, **50**, **48**, **46** may be routed in any equivalent manner throughout the access port housing **34.** The fluid conduits **44**, **50**, **48**, **46** may link to the tube **15** and/or the coupler **42** in a manner to produce equivalent structural and operative configurations as shown, for example, in FIGS. 8A and 8B. In one embodiment, the asymmetric flow regulator **20** may be positioned to a side of the fluid chamber **36**. In one embodiment, the size and configuration of the shunt valve **38** may be varied as desired to produce equivalent results. For example, the shunt valve **38** may be structured such that the plate **88** pivots or bends in response to the force exerted by the syringe **26**.

In one embodiment, the asymmetric flow regulator **20** may be equivalently replaced by a single valve device. In this embodiment, the single valve device would operate to provide the functions of both the one-way valve **52** and the flow control valve **54,** as discussed in relation to FIG. 5B. For example, the single valve device may comprise a valve capable of only allowing a fast flow rate in a first direction when incident fluid pressure exceeds a threshold value. In addition, the single valve device may include a flow restriction device that allows flow at a slow rate in both the first direction, and in a second, opposite direction. The flow restriction device would allow flow from the reservoir to the gastric band. Thus, the single valve device may reproduce the functions of both the one-way valve **52** and the flow control valve **54**, yet would be integrated into a single component, unlike the embodiment of the asymmetric flow regulator **20** shown, for example, in FIG. 5B, in which the flow restriction device comprises a device being separate from the valve configured to open when a threshold pressure has been exceeded.

In one embodiment, the flow restriction device may be configured as a device that only allows flow in one direction of: from the reservoir **18** to the inflatable member **24.** In this embodiment, the flow restriction device may not allow fluid to pass from the inflatable member **24** to the reservoir **18.** The flow restriction device in this embodiment may serve as a return valve, to only allow fluid flow to slowly pass from the reservoir **18** to the inflatable member **24,** to return the inflatable member **24** to a constricted state.

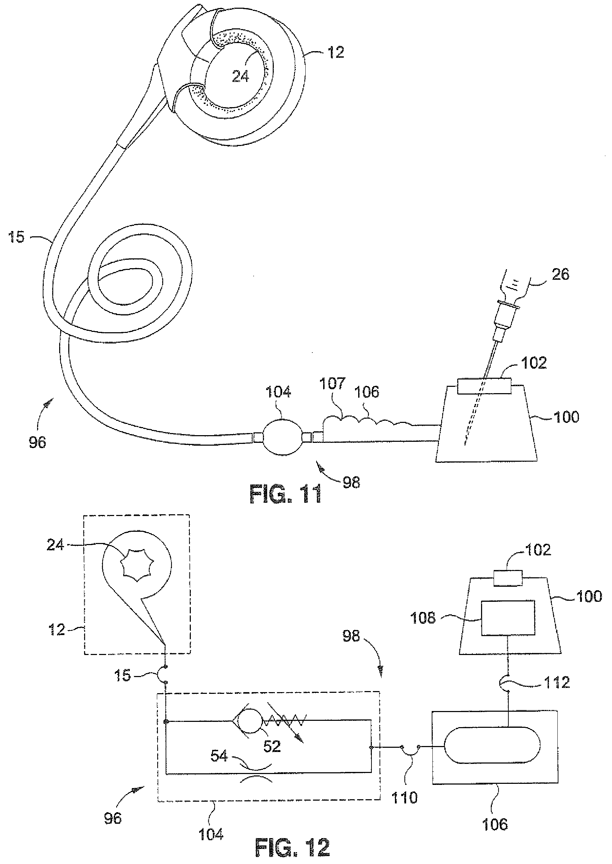

FIG. 11 illustrates an embodiment of a gastric banding system **96** of the present invention, including a gastric band **12,** a tube **15,** an adjustment system **98** and an access port **100.** The access port **100** comprises a standard access port **100** including a septum **102** for receiving the syringe **26**. The adjustment system **98** includes an asymmetric flow regulator **104** and a reservoir **106**. The reservoir **106** couples to the access port **100** in-line, or in series with the asymmetric flow regulator **104.** The tube **15** couples the asymmetric flow regulator **104** to the gastric band **12**.

The reservoir **106** is configured similarly as the reservoir **18** shown, for example, in FIGS. 5A and 5B. The reservoir **106** may comprise a flexible balloon, or bladder, capable of expanding or reducing in size in response to a volume of fluid contained within the reservoir **106**. The reservoir **106** is positioned outside the access port **100** to allow the reservoir to expand without being limited by the dimensions of the access port **100.** One end of the reservoir **106** is coupled to the access port, and the other end is coupled to asymmetric flow regulator **104.** The reservoir **106** has two openings, or two inlet/outlets. The reservoir **106** may include a ribbed or ridged structure **107,** including a plurality of notches on the surface of the reservoir 106. The notches enhance the flexibility of the reservoir **106** by forming pre-stressed regions of the reservoir **106.**

The asymmetric flow regulator **104** is configured similarly as the asymmetric flow regulator **20** shown, for example, in FIG. 5B. However, in this embodiment, the asymmetric flow regulator **104** is not integrated within an access port, but is positioned outside of the access port. One end of the asymmetric flow regulator **104** couples to the reservoir **106** and the other end couples to the tube **15.** The access port **100,** the reservoir **106,** the asymmetric flow regulator **104,** the tube **15** and inflatable member **24** of the gastric band **12** are all in serial fluid communication with each other.

FIG. 12 illustrates a schematic representation of the gastric banding system **96** shown in FIG. 11. The asymmetric flow regulator **104** includes the valve, capable of opening when the incident fluid pressure exceeds a threshold value, shown in FIG. 12 as a one-way valve **52,** and a flow restriction device, shown in FIG. 12, as a flow control valve **54.** The one-way valve **52** and the flow control valve **54,** are configured in the manner discussed, for example, in relation to FIG. 5B. The one-way valve **52** is configured to block fluid flow passing from the reservoir **106** to the inflatable member **24**, and to only allow fluid to pass from the inflatable member **24** to the reservoir **106** when the incident fluid pressure exceeds a threshold value. The flow control valve **54** is configured to allow fluid flow at a slow flow rate between the reservoir **106** and the inflatable member **24**. The one-way valve **52** and the flow control valve **54** may be contained within a shell structure that contains fluid conduits, configured to route fluid in the manner shown in the schematic of FIG. 12.

A coupler **110** links the asymmetric flow regulator **104** to the reservoir **106.** A coupler **112** links the reservoir **106** to a fluid chamber **108** of the access port **100.** The couplers **110,** 112, may be configured to allow components of the gastric banding system **96** to detach from each other. For example, the reservoir **106** may be detachably coupled to the access port **100**, and detachably coupled to the asymmetric flow regulator **104.** Further, the asymmetric flow regulator **104** may be detachably coupled to the tube **15.**

The gastric banding system **96** shown in FIGS. 11 and 12 beneficially allows for a simple design and construction of the adjustment system **98.** The access port **100** may comprise a standard access port, which does not require integration of an asymmetric flow regulator **104**. The asymmetric flow regulator **104** and reservoir **106** are positioned entirely outside of the access port **100.** A standard gastric banding system comprising a band, and a tube coupled to an access port may then be easily modified. For example, referring to a system in which the tube **15** were directly coupled to the access port **100,** a user could remove the tube **15** from the access port **100,** and then couple the reservoir **106** to the outlet of the access port **100.** The user would then couple the asymmetric flow regulator **104** to the reservoir **106.** The user would then couple the tube **15** to the asymmetric flow regulator **104** to connect the gastric band **12** to the access port **100**. The components of the adjustment system 98 could be removed from the tube **15** and access port **100** by performing these steps in reverse.

The system **96** shown in FIGS. 11 and 12 also beneficially allows for a streamlined, or in-line, position of the reservoir 106, as opposed to the position of the reservoir **18** shown, for example, in FIG. 5A. The streamlined position of the reservoir 106 allows for ease of extraction of the reservoir **106** if the gastric banding system **96** were removed from the patient's body.

A drawback to the embodiment shown in FIGS. 11 and 12 is that a shunt valve, for example the shunt valve **38** shown, for example, in FIG. 8B, may not be easily integrated across the asymmetric flow regulator **104.** To adjust the volume of fluid in the system **96,** a physician would access the access port **100** by inserting a syringe **26** needle into the fluid chamber **108**. If fluid is being introduced into the system **96**, then fluid will pass through the reservoir **106,** and through the flow control valve **54,** before it reaches the inflatable member **24.** The restriction caused by the flow control valve **54** may cause a delay effect between the fluid filling the reservoir **106** and the fluid filling the inflatable member **24.** The physician may then have to wait for the pressures of the inflatable member **24** and the reservoir **106** to equalize before the physician can determine if the size of the gastric band **12** has been properly adjusted. In addition, if the physician utilizes a syringe having an integrated pressure sensor, then the physician may have to wait, before receiving a proper pressure signal for the gastric banding system **96**.

In one embodiment, the reservoir **106** and the access port **100** may be combined as a single unit. The access port **100** may not be necessary for operation of the system **96**, if the reservoir **106** is configured to receive a syringe capable of transferring fluid to and from the reservoir **106**. In this embodiment, the reservoir **106** may be appropriately fixed to a portion of the patient's body, in a position that is accessible by a syringe.

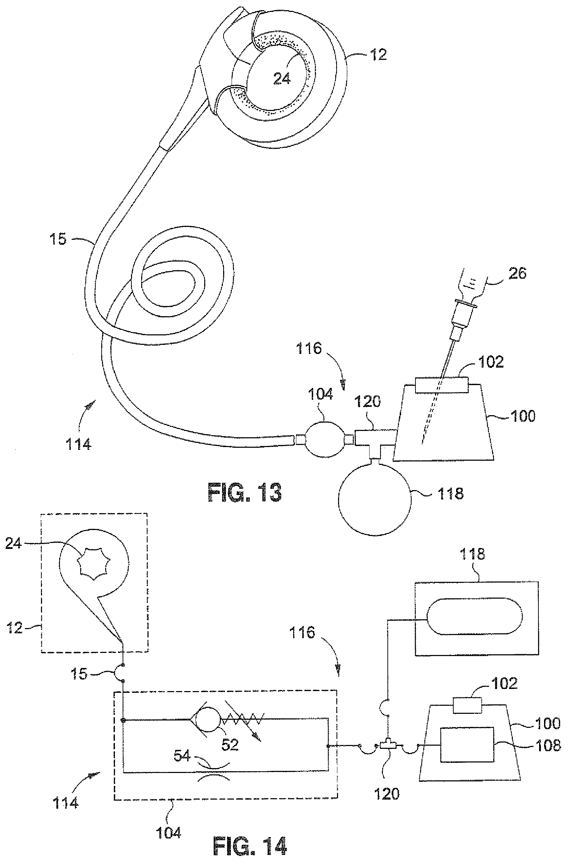

FIG. 13 illustrates an embodiment of a gastric banding system **114** of the present invention including the gastric band **12,** the tube **15**, an adjustment system **116,** and the access port **100.** The adjustment system **116** includes the asymmetric flow regulator **104** and a reservoir **118.** The reservoir **118** couples to the access port **100** through a t-connector **120,** or in parallel with the asymmetric flow regulator **104.** The tube **15** couples the asymmetric flow regulator **104** to the gastric band **12**.

The reservoir **118** is configured similarly as the reservoir **18** shown, for example, in FIGS. 5A and 5B. The reservoir **118** may comprise a flexible balloon, or bladder, capable of expanding or reducing in size in response to a volume of fluid contained within the reservoir **118.** The reservoir **118** may be configured to have only one inlet/outlet, which may reduce the complexity of the reservoir **118,** as opposed to a reservoir having two inlet/outlets, as shown, for example, in FIG. 12. The reservoir **118** is positioned outside the access port **100** to allow the reservoir **118** to expand without being limited by the dimensions of the access port **100.**

The asymmetric flow regulator **104** is configured similarly as the asymmetric flow regulator **104** shown, for example, in FIGS 11 and 12. The access port **100**, the reservoir **118**, the asymmetric flow regulator **104,** the tube **15** and inflatable member **24** of the gastric band **12** are all in fluid communication with each other.

FIG. 14 illustrates a schematic representation of the gastric banding system **114** shown in FIG. 13. The asymmetric flow regulator **104** is configured similarly as the asymmetric flow regulator **104** shown in FIG. 12. For example, the one-way valve **52** and flow control valve **54** may be contained within a shell structure, containing fluid conduits configured to route fluid in the manner shown in the schematic of FIG. **14****.**

The t-connector **120** couples the asymmetric flow regulator **104** and the access port **100** to the reservoir **118**. The t-connector **120** may be configured to allow the asymmetric flow regulator **104** and the access port **100** and the reservoir **118** to detach from each other.

The gastric banding system **114** shown in FIGS. 13 and 14 operates similarly as the system **96** shown in FIGS. 11 and 12. However, in this embodiment, the reservoir **118** is not linked in series with the asymmetric flow regulator **104** and the access port **100.** If fluid is introduced into the access port **100,** it does not have to pass through the reservoir **118** before reaching the inflatable member **24.** Further, the embodiment allows for a more simplistic reservoir **118** design, which only includes one inlet/outlet, as opposed to the two inlet/outlets of the reservoir **106** shown in FIGS. 11 and 12. The gastric banding system **114** additionally includes the drawback that a shunt valve, for example the shunt valve **38** shown, for example, in FIG. 8B, may not be easily integrated across the asymmetric flow regulator **104**.

## Claims

1. A system (10) for constricting a stomach (22) of a patient for treating obesity, the system comprising:
a gastric band (12) having an inflatable member (24) configured to contain fluid and apply constriction to a portion of a gastric lumen of the stomach, the inflatable member (24) being moveable from a constricted state to a passage state for allowing an obstruction to pass through the portion of the gastric lumen, the passage state being when less fluid is contained in the inflatable member (24) than in the constricted state;
a valve (52) configured to move from a closed position to an open position when a pressure of the fluid from the inflatable member (24) increases over a threshold in response to the obstruction passing through the gastric lumen, and to allow the fluid from the inflatable member (24) to pass through the valve (52) at a first flow rate;
a reservoir (18, 106) configured to receive the fluid passed through the valve (52), allowing the inflatable member (24) to move from the constricted state to the passage state, and allowing the obstruction to pass through the portion of the gastric lumen; and
a flow restriction device (54) configured to allow the fluid received by the reservoir (18, 106) to pass from the reservoir (18, 106) to the inflatable member (24) at a second flow rate, allowing the inflatable member (24) to return to the constricted state,
**characterized in that** the second flow rate is less than the first flow rate.

2. The system of claim 1 wherein the flow restriction device (54) comprises a device being separate from the valve (52).

3. The system of claim 1 or 2 wherein the flow restriction device (54) is configured to allow fluid from the inflatable member (24) to pass to the reservoir (18, 106).

4. The system of any of the preceding claims wherein the valve (52) is a one-way valve configured to only allow fluid to pass through the one-way valve in a flow direction of: from the inflatable member (24) to the reservoir (18, 106).

5. The system of any of the preceding claims wherein the threshold is defined by a structural configuration of the valve (52), optionally wherein the threshold is variable.

6. The system of any of the preceding claims wherein the flow restriction device (54) is a flow control valve.

7. The system of any of the preceding claims wherein the flow restriction device (54) is configured such that the second flow rate is variable.

8. The system of any of the preceding claims wherein the valve (52) and the flow restriction device (54) are in fluid communication with the reservoir (18, 106) in a parallel configuration.

9. The system of any of the preceding claims wherein the valve (52) and the flow restriction device (54) are in direct fluid communication with the reservoir (18, 106).

10. The system of any of the preceding claims wherein the valve (52) is in direct fluid communication with the flow restriction device (54).

11. The system of any of the preceding claims wherein the reservoir (18, 106) is configured such that a volume of the reservoir (18, 106) increases automatically when the reservoir (18, 106) receives the fluid passed through the valve (52), optionally wherein the reservoir (18, 106) comprises a flexible bladder.

12. The system of any of the preceding claims wherein the valve (52) is configured to open automatically when a pressure of the fluid from the inflatable member (24) exceeds the threshold.

13. The system of any of the preceding claims wherein the gastric band (12) is configured to encircle the portion of the gastric lumen of the stomach (22) to form a diameter, the diameter having a first size when the inflatable member (24) is in the passage state, and having a second size when the inflatable member (24) is in the constricted state, the first size being larger than the second size.

14. The system of any of the preceding claims further comprising an access port (16), wherein the valve (52) and the flow restriction device (54) are positioned within the access port (16), optionally further comprising a shunt valve (38) configured to open to allow fluid to pass to the reservoir (18, 106) and to the inflatable member (24) without passing through the valve (52) and without passing through the flow restriction device (54), further optionally wherein the shunt valve (38) is in direct fluid communication with the reservoir (18, 106).

## Patentansprüche

1. System (10) zum Einengen eines Magens (22) von einem Patienten zum Behandeln von Fettleibigkeit, das System mit:
einem Magenband (12), das ein aufblasbares Element (24) aufweist, welches eingerichtet ist, Fluid zu enthalten und eine Einengung auf einen Abschnitt eines Magenlumens des Magens aufzubringen, wobei das aufblasbare Element (24) von einem eingeengten Zustand zu einem Durchgangszustand bewegbar ist, um zuzulassen, dass eine Obstruktion durch den Abschnitt des Magenlumens gelangt, und der Durchgangszustand vorliegt, wenn weniger Fluid in dem aufblasbaren Element (24) als in dem eingeengten Zustand enthalten ist;
einem Ventil (52), das eingerichtet ist, sich von einer geschlossenen Position zu einer offenen Position zu bewegen, wenn ein Druck des Fluids von dem aufblasbaren Element (24) in Antwort auf das hindurch Gelangen der Obstruktion durch das Magenlumen über einen Grenzwert steigt, und um zuzulassen, dass Fluid von dem aufblasbaren Element (24) durch das Ventil (52) mit einer ersten Strömungsgeschwindigkeit gelangt;
einem Reservoir (18, 106), das eingerichtet ist, das durch das Ventil (52) gelangte Fluid zu empfangen, was zulässt, dass sich das aufblasbare Element (24) von dem eingeengten Zustand zu dem Durchgangszustand bewegt und die Obstruktion durch den Abschnitt des Magenlumens gelangt; und
einer Strömungsbeschränkungseinrichtung (54), die eingerichtet ist, zuzulassen, dass das durch das Reservoir (18, 106) empfangene Fluid mit einer zweiten Strömungsgeschwindigkeit von dem Reservoir (18, 106) zu dem aufblasbaren Element (24) gelangt, was zulässt, dass das aufblasbare Element (24) zu dem eingeengten Zustand zurückkehrt,
**dadurch gekennzeichnet, dass** die zweite Strömungsgeschwindigkeit geringer ist als die erste Strömungsgeschwindigkeit.

2. System nach Anspruch 1, bei dem die Strömungsbeschränkungseinrichtung (54) eine Einrichtung aufweist, die von dem Ventil (52) getrennt ist.

3. System nach Anspruch 1 oder 2, bei dem die Strömungsbeschränkungseinrichtung (54) eingerichtet ist, zuzulassen, dass Fluid von dem aufblasbaren Element (24) zu dem Reservoir (18, 106) gelangt.

4. System nach einem der vorstehenden Ansprüche, bei dem das Ventil (52) ein Einwegventil ist, das eingerichtet ist, nur zuzulassen, dass Fluid durch das Einwegventil in einer Strömungsrichtung von dem aufblasbaren Element (24) zu dem Reservoir (18, 106) gelangt.

5. System nach einem der vorstehenden Ansprüche, bei dem der Grenzwert durch eine strukturelle Ausführung des Ventils (52) definiert ist, wobei der Grenzwert optional eine Variable ist.

6. System nach einem der vorstehenden Ansprüche, bei dem die Strömungsbeschränkungseinrichtung (54) ein Strömungssteuerungsventil ist.

7. System nach einem der vorstehenden Ansprüche, bei dem die Strömungsbeschränkungseinrichtung (54) so eingerichtet ist, dass die zweite Strömungsgeschwindigkeit veränderlich ist.

8. System nach einem der vorstehenden Ansprüche, bei dem das Ventil (52) und die Strömungsbeschränkungseinrichtung (54) mit dem Reservoir (18, 106) in einer parallelen Anordnung in Fluidverbindung sind.

9. System nach einem der vorstehenden Ansprüche, bei dem das Ventil (52) und die Strömungsbeschränkungseinrichtung (54) mit dem Reservoir (18, 106) in direkter Fluidverbindung sind.

10. System nach einem der vorstehenden Ansprüche, bei dem das Ventil (52) mit der Strömungsbeschränkungseinrichtung (54) in direkter Fluidverbindung steht.

11. System nach einem der vorstehenden Ansprüche, bei dem das Reservoir (18, 106) so eingerichtet ist, dass ein Volumen des Reservoirs (18, 106) automatisch ansteigt, wenn das Reservoir (18, 106) das durch das Ventil (52) gelangte Fluid empfängt, wobei das Reservoir (18, 106) optional eine elastische Blase aufweist.

12. System nach einem der vorstehenden Ansprüche, bei dem das Ventil (52) eingerichtet ist, sich automatisch zu öffnen, wenn ein Fluiddruck von dem aufblasbaren Element (24) den Grenzwert übersteigt.

13. System nach einem der vorstehenden Ansprüche, bei dem das Magenband (12) eingerichtet ist, den Abschnitt des Magenlumens von dem Magen (22) zu umgeben, um einen Durchmesser auszubilden, wobei der Durchmesser eine erste Größe aufweist, wenn das aufblasbare Element (24) in dem Durchgangszustand ist, und eine zweite Größe aufweist, wenn das aufblasbare Element (24) in dem eingeengten Zustand ist, wobei die erste Größe größer ist als die zweite Größe.

14. System nach einem der vorstehenden Ansprüche, ferner mit einer Zugangsöffnung (16), wobei das Ventil (52) und die Strömungsbeschränkungseinrichtung (54) in der Zugangsöffnung (16) positioniert sind, und optional ferner mit einem Shunt-Ventil (38), das eingerichtet ist, sich zu öffnen, um zuzulassen, dass Fluid zu dem Reservoir (18, 106) und zu dem aufblasbaren Element (24) gelangt, ohne durch das Ventil (52) zu gelangen und ohne durch die Strömungsbeschränkungseinrichtung (54) zu gelangen, und wobei das Shunt-Ventil (38) ferner optional mit dem Reservoir (18, 106) in direkter Fluidverbindung steht.

## Revendications

1. Système (10) de rétrécissement de l'estomac (22) d'un patient destiné au traitement de l'obésité, le système comprenant :
un anneau gastrique (12) comportant un élément gonflable (24) conçu pour contenir un liquide et appliquer un rétrécissement à une partie d'une lumière gastrique de l'estomac, l'élément gonflable (24) pouvant passer d'un état rétréci à un état de passage pour permettre à une obstruction de passer par la partie de la lumière gastrique, l'état de passage étant celui dans lequel l'élément gonflable (24) contient moins de liquide qu'à l'état rétréci ;
une valve (52) conçue pour passer d'une position fermée à une position ouverte lorsque la pression du liquide provenant de l'élément gonflable (24) augmente au-delà d'un seuil en réponse au passage de l'obstruction par la lumière gastrique, et pour permettre au liquide provenant de l'élément gonflable (24) de passer par la valve (52) à un premier débit ;
un réservoir (18, 106) conçu pour recevoir le liquide qui est passé par la valve (52), permettant à l'élément gonflable (24) de passer de l'état rétréci à l'état de passage, et permettant à l'obstruction de passer par la partie de la lumière gastrique ; et
un dispositif de limitation de débit (54) conçu pour permettre au liquide reçu par le réservoir (18, 106) de passer du réservoir (18, 106) à l'élément gonflable (24) à un second débit, permettant à l'élément gonflable (24) de revenir à l'état rétréci,
**caractérisé en ce que** le second débit est inférieur au premier débit.

2. Système selon la revendication 1, dans lequel le dispositif de limitation de débit (54) comprend un dispositif qui est indépendant de la valve (52).

3. Système selon les revendications 1 ou 2, dans lequel le dispositif de limitation de débit (54) est conçu pour permettre au liquide provenant de l'élément gonflable (24) de passer dans le réservoir (18, 106).

4. Système selon l'une quelconque des revendications précédentes, dans lequel la valve (52) est un clapet antiretour configuré pour laisser un liquide passer par le clapet antiretour uniquement dans le sens d'écoulement de l'élément gonflable (24) au réservoir (18, 106).

5. Système selon l'une quelconque des revendications précédentes, dans lequel le seuil est défini par la configuration structurelle de la valve (52), éventuellement dans lequel le seuil est variable.

6. Système selon l'une quelconque des revendications précédentes, dans lequel le dispositif de limitation de débit (54) est un régulateur de débit.

7. Système selon l'une quelconque des revendications précédentes, dans lequel le dispositif de limitation de débit (54) est configuré de telle sorte que le second débit soit variable.

8. Système selon l'une quelconque des revendications précédentes, dans lequel la valve (52) et le dispositif de limitation de débit (54) sont en communication fluidique avec le réservoir (18, 106) dans une configuration parallèle.

9. Système selon l'une quelconque des revendications précédentes, dans lequel la valve (52) et le dispositif de limitation de débit (54) sont en communication fluidique directe avec le réservoir (18, 106).

10. Système selon l'une quelconque des revendications précédentes, dans lequel la valve (52) est en communication fluidique directe avec le dispositif de limitation de débit (54).

11. Système selon l'une quelconque des revendications précédentes, dans lequel le réservoir (18, 106) est configuré de telle sorte que le volume du réservoir (18, 106) augmente automatiquement lorsque le réservoir (18, 106) reçoit le liquide qui est passé par la valve (52), éventuellement dans lequel le réservoir (18, 106) comprend une vessie souple.

12. Système selon l'une quelconque des revendications précédentes, dans lequel la valve (52) est configurée pour s'ouvrir automatiquement lorsque la pression du liquide provenant de l'élément gonflable (24) dépasse le seuil.

13. Système selon l'une quelconque des revendications précédentes, dans lequel l'anneau gastrique (12) est conçu pour enserrer la partie de la lumière gastrique de l'estomac (22) pour former un diamètre, le diamètre ayant une première dimension lorsque l'élément gonflable (24) est à l'état de passage et ayant une seconde dimension lorsque l'élément gonflable (24) est à l'état rétréci, la première dimension étant supérieure à la seconde dimension.

14. Système selon l'une quelconque des revendications précédentes, comprenant en outre un orifice d'accès (16), dans lequel la valve (52) et le dispositif de limitation de débit (54) sont placés dans l'orifice d'accès (16), comprenant éventuellement en outre une valve de dérivation (38) configurée pour s'ouvrir afin de laisser le liquide passer jusqu'au réservoir (18, 106) et jusqu'à l'élément gonflable (24) sans passer par la valve (52) et sans passer par le dispositif de limitation de débit (54), éventuellement en outre dans lequel la valve de dérivation (38) est en communication fluidique directe avec le réservoir (18, 106).
